# EUROPÄISCHE PATENTANMELDUNG

(11) **EP 2 233 162 A2**
(43) Veröffentlichungstag der Anmeldung: **29.09.2010**
(21) Anmeldenummer: 10155867.4
(22) Anmeldetag: 09.03.2010
(51) Int. Cl.: A61L 31/02, A61L 31/08, A61L 31/14

(54) **Medizinisches Implantat zur Medikamentenfreisetzung mit poröser Oberfläche**

(30) Priorität: 26.03.2009 DE 102009001895
(71) Anmelder: Biotronik VI Patent AG, 6341 Baar (CH)
(72) Erfinder: Sager, Laura, 8038, Zürich (CH); Klocke, Björn, 8006, Zürich (CH); Müller, Heinz, 91052, Erlangen (DE); Adden, Nina, 90427, Nürnberg (DE); Kappelt, Gerhard, 91054, Erlangen (DE)
(74) Vertreter: Lindner-Vogt, Karin L.

(57) **Zusammenfassung**

Es wird ein Implantat vorgeschlagen, das insbesondere aus einem Grundmaterial hergestellt ist, welches aus einem biologisch abbaubaren Metall und/oder einer biologisch abbaubaren Metalllegierung besteht, wobei das Implantat eine Beschichtung aufweist, die aus kristallinem Calciumphosphat und/oder amorphem Calciumphosphat besteht.

## Beschreibung

Die Erfindung betrifft ein medizinisches Implantat, vorzugsweise einen absorbierbaren Metallstent (AMS) mit einer beschichteten Oberfläche.

Arteriosklerose ist eine weit verbreitete Krankheit der menschlichen Arterien. Verschiedene Arten von Substanzen, die Plaque genannt werden, sammeln sich an den Wänden der Arterien und schränken den natürlichen Blutdurchfluss ein. Wenn die Krankheit fortschreitet, besteht die Gefahr, dass bei Patienten durch Ischämie Fehlfunktionen von Teilen des Körpers auftreten, die über das entsprechende Gefäß mit Blut versorgt werden.

Für Plaqueläsionen gibt es verschiedene Formen der Behandlung, wie zum Beispiel Bypass-Chirurgie und Arterektomie. Die am weitesten verbreiteten Behandlungsformen sind die nichtinvasiven Verfahren mit Ballonkathetern zum Öffnen der Läsion und Einsetzen eines Stents zum Abstützen der wiedereröffneten Arterie und Verhindern des erneuten Zusammenfallens des Gefäßes.

Stents werden üblicherweise aus beständigen Materialien hergestellt, wie zum Beispiel 316L und Nitinol. Im Laufe der letzten Jahre wurden abbaubare Stents entwickelt, die biologisch absorbiert werden, wenn die Gerüstfunktion des Stents nicht mehr benötigt wird. Es wurden insbesondere Stents aus Magnesiumlegierungen entwickelt und klinisch in den Koronarien untersucht. Es ist gezeigt worden, dass diese Stents sicher sind, aber abgebaut werden, bevor die Tendenz des Gefäßes zum Wiederzusammenfallen aufhört. Daher litten diese Stents am Wiederzusammenfallen der Gefäße (wie es vorher bei der reinen Ballon-Angioplastie ohne Stentverwendung bekannt war). Es konnte auch gezeigt werden, dass es eine Bildung von Gewebe im Innern des Stents (Neointimaproliferation) gibt, die auch von nicht abbaubaren Stents bekannt ist.

Klinisch erfolgreiche Stents aus Magnesiumlegierung müssen eine längere Stützfähigkeit als heute bekannt aufweisen und profitieren auch von einer reduzierten Neointimaproliferation.

Zur Verringerung des Abbaus der Stents aus Magnesiumlegierung und damit zur Verlängerung der Stützzeit sind Modifizierungen der Oberfläche vorgeschlagen worden. Dazu gehören anorganische Beschichtungen, die aus Phosphaten, Oxiden und anderen Stoffen bestehen. Experimente haben gezeigt, dass diese Beschichtungen bei der Verringerung der Abbauraten von unverformten Proben wirksam sind. Stents, die jedoch vor der Implantation auf einen Katheter gecrimpt und am Behandlungsort im menschlichen Körper dilatiert werden, erfahren eine starke Oberflächenverformung (normalerweise 20-40%) des metallischen Stentkörpers, und die anorganische Beschichtung bekommt Risse und wird spröde, was den Effekt der Abbaukontrolle unwirksam macht.

Eines der bekannten anorganischen Beschichtungsmaterialien ist Hydroxyapatit. Heute ist Hydroxyapatit als Implantatsmaterial gut bekannt. Es weist eine hervorragende Bioverträglichkeit beim Kontakt mit den meisten Arten von menschlichem Gewebe auf. Daher hat Hydroxyapatit einen großen Anwendungsbereich in der Implantologie gefunden, meistens als Beschichtung und meistens für orthopädische oder traumatologische Implantate. Bei diesen Anwendungen können die Implantatsoberflächen durch viele Ablagerungsprozesse, wie zum Beispiel durch Plasmaspritzprozesse, chemische Aufdampfungsprozesse, Sinterprozesse, elektrochemische Prozesse und viele andere, mit Hydroxyapatit beschichtet werden. Diese Prozesse scheiden die Hydroxapatitschichten auf der ursprünglichen Implantatsoberfläche oder auf einer Grundierungsschicht ab. Leider hat sich herausgestellt, dass Hydroxyapatitschichten bei der Beherrschung des Stentabbaus unwirksam sind, weil sie nicht die hochelastische Verformung aufweisen, die typischerweise für Stents benötigt wird.

Die Kontrolle der Neointimaprofileration für Permanent-Stents wird normalerweise durch Medikamente freisetzende Beschichtungen auf den Stents erreicht. Normalerweise werden antiproliferativ wirkende Medikamente, wie zum Beispiel Paclitaxel oder Sirolimus und ihre Derivate, eingebettet aufgebracht, d.h. mit Trägersubstanzen aus beständigen Polymeren (wie zum Beispiel Polysulphon und SIBS) oder abbaubaren Polymeren (Polyester, wie zum Beispiel PLLA, PCL und PLGA) vermischt. Diese Verfahren können jedoch nicht für Stents aus Magnesiumlegierung angewendet werden. Beständige Polymere werden durch die Wasserstoffentwicklung aus Magnesiumoberflächen aufgebläht, und abbaubare Polyester beschleunigen den Magnesiumabbauprozess durch ihren sauren Zerfall in ungünstiger Weise. Es sind speziellere, abbaubare, Medikamente freisetzende Beschichtungen, die nicht vom Polyestertyp sind, vorgeschlagen worden. Diesen Beschichtungen fehlt aber normalerweise die Haftfähigkeit an Magnesiumoberflächen.

Nachteile der bekannten Konzepte für einen Medikamente freisetzenden, absorbierbaren Metallstent (Drug Eluting Absorbable Metal Stent = DREAMS) sind:
- DREAMS mit normalem beständigem Medikamententräger: kein vollständiger Abbau des Stents, Probleme bei Wasserstoffentwicklung (obwohl die Wasserstoffentwicklung sich nicht auf die Beschichtung, sondern auf das Grundmaterial bezieht).
- DREAMS mit normalem, abbaubarem Medikamententräger auf Polymerbasis (PLLA, PLGA usw. und Polyester): ungünstige biologische Wechselwirkung, Anschwellen des Polymers, daher kein langzeitiger Schutz von Mg gegen Körperflüssigkeiten, selbst bei abbaubaren Polymeren keine nachgewiesene Sicherheit gegen subakute Thrombosen, Probleme mit dem sauren Zerfall der Polyester
- DREAMS mit Lipidmedikamententräger: mangelhaftes Haften des (weichen/ziemlich flüssigen) Trägers, oft zu schnelle Freisetzung
- DREAMS mit poröser Oberfläche und Medikament darin: zu schnelle Freisetzungskinetik

Das Ziel der vorliegenden Erfindung ist es, diese Nachteile zu beseitigen.

Dieses Ziel wird durch ein medizinisches Implantat, wie zum Beispiel einen Stent, erreicht, der aus einem Grundmaterial hergestellt ist, das aus einem biologisch abbaubaren Metall und/oder einer biologisch abbaubaren Metalllegierung besteht, wobei das Implantat eine Beschichtung umfasst, die aus einem kristallinen Calciumphosphat und/oder amorphem Calciumphosphat hergestellt ist.

Die Calciumphosphatbeschichtung kann durch eine Beschichtung mit Hydroxyapatit, einem biologischen Apatit, Dicalciumphosphaten, Tricalciumphosphaten, Fluorapatit, Chlorapatit oder einer Verbindung erreicht werden, die hauptsächlich aus Calciumphosphat in einer Hydroxyapatit ähnlichen oder Hydroxyapatit nahe kommenden stöchiometrischen Zusammensetzung besteht.

Die erwähnte Beschichtung des Implantats kann Spuren oder Abscheidungen des Grundmaterials des Implantats enthalten. Das Grundmaterial ist Magnesium oder eine Magnesiumlegierung, vorzugsweise ist die Magnesiumlegierung WE43.

Die Magnesiumlegierung kann ein Legierungselement von bis zu 50 Atomprozent aus der Gruppe bestehend aus Calcium, Zink, Mangan, Lithium, Eisen oder bis zu 50 Atomprozent einer Kombination derselben enthalten.

Die Magnesiumlegierung kann zusätzlich bis zu 20 Atomprozent Yttrium oder andere Seltenerdelemente enthalten. in diesem Fall bestehen die Abscheidungen aus Yttrium und/oder einer anderen Seltenen Erde, wie zum Beispiel Neodym. Die Abscheidungen haben zum Beispiel eine Größe von 0,5-3 Mikrometern für Yttrium und 1-7 Mikrometern für Neodym.

In einer bevorzugten Ausführungsform hat die Calciumphosphatbeschichtung eine Schichtstruktur. Die erste Schicht, die direkten Kontakt mit dem medizinischen Implantat hat, ist eine kristallisierte Calciumphosphatschicht. Das ist eine in hohem Maße dichte Schicht, die den Abbau des darunter liegenden abbaubaren Materials des Implantats steuert und verzögert. Auf diese kristalline, sehr dichte Schicht wird eine amorphe Calciumphosphatschicht aufgetragen.

Mindestens eine der Schichten der Beschichtung hat vorzugsweise eine Schichtdicke von 0,1 bis 5 Mikrometern.

In einer bevorzugten Ausführungsform hat die amorphe Calciumphosphatschicht Poren mit einer Porengröße zwischen 0,02 und 50 Mikrometern. Falls die poröse Schicht aus Hydroxyapatit besteht, liegt die Porengröße vorzugsweise im Bereich von 0,02 bis 1 Mikrometer.

Die poröse Schicht des Implantats kann mit einem oder mehreren Lipiden gefüllt sein, vorzugsweise Fettsäure, Mono-, Di- oder Triacylglyceriden, Wachsen, Phospholipiden, Sphingolipiden, Steroiden und abbaubaren Polymeren, die einen nichtsauren Zerfallsmechanismus zeigen.

Außerdem kann ein Medikament entweder in die amorphe Calciumphosphatschicht oder in die Lipidkomponente eingebettet sein. Das Medikament oder die Medikamente können entzündungshemmende und/oder antiproliferative Medikamente sein, einschließlich Paclitaxel, Sirolimus und ihre Analoga, und/oder antimykotische Medikamente sein usw.. Auch die Verwendung von mehreren Medikamenten ist möglich.

Das antiproliferative, entzündungshemmende und/oder antimykotische Medikament kann aus der folgenden Liste ausgewählt werden: Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanol A,B und C, Bryophyllin A, Busulfan, Antithrombin, Bivalirudin, Cadherin, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Interieuretid (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilon A and B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, [beta]-myc-Antisense, [beta]-Lapachon, Podophyllotoxin, Podophyllsäure-2-ethylhydrazid, Molgramostim (rhuGM-CSF), Peginterferon [alpha]-2b, Lanograstim (r-HuG-CSF), Macrogol, Selectin (Cytokinantagonist), Cytokininhibitoren, COX-2-Inhibitor, NFkB, Angiopeptin, monoclonale Antikörper, die die Muskezellproliferation blockieren, bFGF-Antagonisten, Probucol, Prostaglandine, 1-Hydroxy-11-Methoxycanthin-6-on, Scopolectin, NO-Donor, wie Pentaerythrityltetranitrat und Syndnoeimin, S-Nitrosoderivat, Tamoxifen, Staurosporin, [Beta]-Estradiol, [Alpha]-Estradiol, Estriol, Estron, Ethinylestradiol, Medroxyprogesteron, Estradiolcypionat, Estradiolbenzoat, Tranilast, Kamebakaurin und andere Terpenoide, die in der Krebstherapie verwendet werden, Verapamil, Tyrosin-Kinase-Inhibitoren (Tyrphostine), Paclitaxel und ihre Derivate, wie 6-[Alpha]-Hydroxy-Paclitaxel, Taxotere, Kohlensuboxide (MCS) und ihre macrozyklischen Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, [Beta]-Sitosterin, Myrtecain, Polidocanol, Nonivamid, Levomenthol, Ellipticin, D-24851 (Calbiochem), Colcemid, Cytochalasin A-E, Indanocin, Nocadazol, S 100 Protein, Bacitracin, Vitronectin-Rezeptor-Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plasminogen-Aktivator Inhibitor-1, Plasminogen-Aktivator Inhibitor-2, Antisense-Oligonucleotid, VEGF-Inhibitoren, IGF-1, Medikamente aus der Gruppe der Antibiotika, wie Cefadroxil, Cefazolin, Cefaclor, Cefotixin, Tobramycin, Gentamycin, Penicillin, wie Dicloxacillin, Oxacillin, Sulfonamide, Metronidazol, Enoxoparin, desulfatiertes und N-reacetyliertes Heparin (Hemoparin<(R)>), Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren, wie Dipyramidol, Trapidil, Nitroprussid, PDGF-Antagonisten, wie Triazolopyrimidin und Seramin, ACE-Inhibitoren, wie Captopril, Cilazapril, Lisinopril, Enalapril, Losartan, Thioproteaseinhibitoren, Prostacyclin, Vapiprost, Interferon [Alpha], [Beta] und [Gamma], Histaminantagonisten, Serotoninblocker, Apoptoseinhibitoren, Apoptoseregulatoren, wie p65, NF-kB oder Bcl-xL-Antisense-Oligonukleotide, Halofuginon, Nifedipin, Tocopherol Tranilast, Molsidomin, Teepolyphenol, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetische Steroide, wie Inotodiol, Maquirosid A, Ghalakinosid, Mansonin, Streblosid, Hydrocortison, Betamethason, Dexamethason, nonsteroidale Substanzen (NSAIDS), wie Fenoporfen, Ibuprofen, Indomethacin, Naproxen, Phenylbutazon und andere antivirale Agentien, wie Acyclovir, Ganciclovir und Zidovudin, Clotrimazol, Flucytosin, Griseofulvin, Ketoconazol, Miconazol, Nystatin, Terbinafin, antiprotozoale Agentien, wie Chloroquin, Mefloquin, Quinin, natürliches Terpenoid, wie Hippocaesculin, Barringtogenol-C21-Angelat, 14-Dehydroagrostistachin, Agroskerin, Agrostistachin, 17-Hydroxyagrostistachin, Ovatodiolid, 4,7-Oxycycloanisomelsäure, Baccharinoide B1, B2, B3 und B7, Tubeimosid, Bruceantinoside C, Yadanzioside N und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A and B, Longikaurin B, Sculponeatin C, Kamebaunin, Leukamenin A and B, 13,18-Dehydro-6-alpha-Senecioyloxychaparrin, Taxamairin A and B, Regenilol, Triptolid, Cymarin, Hydroxyanopterin, Protoanemonin, Cheliburinchlorid, Sinococulin A and B, Dihydronitidin, Nitidinchlorid, 12-beta-Hydroxypregnadien 3,20-dion, Helenalin, Indicin, Indicin-N-oxid, Lasiocarpin, Inotodiol" Podophyllotoxin, Justicidin A and B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A and B, Zeorin.

Das Lipid kann auch zum Einbetten von pH-Puffersubstanzen verwendet werden, um den Abbau zu steuern und/oder das Medikament weiter vor hohen pH-Werten zu schützen, die durch die Mg-Zersetzung erzeugt werden.

Biologisch abbaubare metallische Implantate besitzen günstige mechanische und biologische Eigenschaften, neigen aber zum zu schnellen Erodieren, was zu einer vollständigen oder nahezu vollständigen Zersetzung vor dem Ende der Behandlungszeit führt. Das Ziel der vorliegenden Erfindung ist es, eine Beschichtung zur Steuerung einer solchen Zersetzungsrate bereitzustellen. Eine weitere Ausführungsform der vorliegenden Erfindung richtet sich auf ein therapeutisches Mittel, das in die Beschichtung dieser medizinischen Vorrichtung integriert werden kann.

In der Vergangenheit sind Polymere als Beschichtung zur Steuerung des Abbaus von biologisch abbaubaren Stents verwendet worden. Bei der Verwendung von Polymeren tritt beim Abbauprozess oft eine entzündliche Reaktion des Gewebes auf. Hydroxyapatitbeschichtungen als eine Art von Calciumphosphat sind **dadurch gekennzeichnet, dass** sie vollständig biologisch kompatibel sind.

Selbst biologisch abbaubare Metallimplantatmaterialien erzeugen Schichten von Reaktions-/Umwandlungsprodukten, die den Zusammensetzungen von Apatiten ähnlich sind, wenn sie sich in physiologischen Lösungen zersetzen. Apatitschichten können sich aus einer Vorreaktion der Implantatsoberfläche in einer physiologischen Lösung oder ähnlichen Lösungen ergeben. Dies ergibt eine besonders hohe Bindungsfestigkeit der Beschichtung auf dem Substrat. Die Hydroxyapatitschichten, die auf diese Weise erzeugt wurden, sind **dadurch gekennzeichnet, dass** sie bestimmte Mengen oder Spuren des früheren Grundmaterials, das umgewandelt wurde, enthalten. Das könnten zum Beispiel Spuren von Magnesium bei Verarbeitung von absorbierbaren Magnesiumimplantaten oder Legierungselementen oder Abscheidungen aus dem Grundmetall, zum Beispiel Seltenerdmetallen, oder Abscheidungen sein, die Seltenerdmetalle enthalten, wenn Implantate, die aus Seltenerden hergestellt sind, die Magnesiumlegierungen enthalten, verarbeitet werden.

Es wurde überraschend festgestellt, dass Calciumphosphatschichten neben der guten Biokompatibilität eine Menge interessanter Eigenschaften haben. So ist festgestellt worden, dass diese Beschichtungen korrosionshemmende Wirkungen haben. Es ist auch festgestellt worden, dass viele der Beschichtungen eine raue, rissige oder poröse Struktur besitzen. Das individuelle Aussehen der Beschichtung hängt vom Prozess, der verwendet wird, und den Parametern dieses Prozesses ab. Ein wichtiger Parameter ist die Kristallinität der Calciumphosphatphase. Stärker kristalline Phasen werden vom Körper langsamer resorbiert. Neben der Beschichtungsdicke stellt daher die Kristallinität eine Möglichkeit dar, die Passivierungswirkung der Calciumphosphatbeschichtung zu steuern.

Bei Implantaten, die eine plastische Verformung erfahren müssen, können spröde Apatitschichten reißen und daher ihre Schutzeigenschaften verlieren und so eine schnelle lokale Korrosion des Substrats ermöglichen. Um solche Wirkungen zu verhindern, können, neben der Verwendung von Filmen mit einer Schichtdicke von weniger als 1 µm, bestimmte organische Substanzen , wie zum Beispiel Lipide (Fette, Öle usw.) oder bestimmte biologisch abbaubare Polymere mit einem nichtsauren Zerfall, zum Aufbau einer elastischen Beschichtung auf der Calciumphosphatbeschichtung verwendet werden, die das Grundmaterial vor der beschleunigten Korrosion oder sogar vor dem Abbau schützt und bestimmte Abbauintervalle für das abbaubare Implantat aufrechterhält. Lipide sind Fettsäuren, Mono, Di- oder Triacylglyceride, Wachse, Phospholipide, Sphingolipide, Steroide, um nur ein paar zu nennen. Lipide besitzen den Vorteil, dass sie stark hydrophob sind, daher quellen sie nicht in Wasser und schützen den AMS länger gegen die Körperflüssigkeit; so wird der Abbau des Stents verzögert. Mit anderen Worten, solche Lipide stellen eine elastische Abdichtung bereit, um Risse im Hydroxyapatit zu verhüten, die eine ungünstige, unkontrollierte Wirkung auf den Stentabbau haben.

Außerdem können solche Beschichtungen aus Fetten oder Ölen oder (biologisch abbaubaren) Polymeren sehr wirksam als Medikamententrägersubstanzen verwendet werden.

Es gibt einige spezielle Vorteile der beschriebenen erfindungsgemäßen Lösung und zwar:
• Verwendung des Bodens der Poren in Verbindung mit der Verwendung von AMS als Stentplattform.
• Verwendung der Schicht nicht nur wegen ihrer Poren und der Medikamentenanlagerung, sondern auch wegen der Eigenschaften bei der Abbausteuerung für die darunter liegende Mg-Stentstruktur.
• Die Beschichtung enthält Spuren vom metallischen Substrat.

Im Folgenden werden andere oder modifizierte Ausführungsformen beschrieben:
AMS auf der Basis von WE 43 mit einer porösen Hydroxyapatitbeschichtung (ca. 0,1-5 µm Poren plus Trennung von 0,1-5 µm mit eingeschränkter Wasserdiffusion), gefüllt mit hydriertem Sojabohnenöl und Paclitaxel (bei 20% TAXUS-Belastung oder etwa 0,2 µg/mm²), wobei Hydroxyapatit Poren ergibt, aber auch zur Trennung der Mg-Oberfläche vom Träger führt (um schädliche Wechselwirkungen zwischen OH- Ionen und

Lipiden und dem Medikament zu minimieren), wobei die Porengröße (etwa 0,02-1 µm) bzw. getrennte Inseln von der gewünschten Freisetzungskinetik abhängen.
- Zusätzliche Deckschicht zum Abdichten der Stentoberfläche und möglicherweise zusätzliche Steuerung der Medikamentenfreisetzung, z.B. Deckschichten, die aus abbaubaren Polymeren bestehen, wie zum Beispiel Polyestern (z.B. PLGA und PLLA).
- Trennschicht statt oder zusätzlich zum gefüllten Boden von Poren zum Trennen von Mg und Lipid/ Medikament (z.B. Siliziumkarbid, diamantenähnlicher Kohlenstoff). Alle anderen abbaubaren Metallstents (einschließlich verschiedener Mg-Legierungen, allgemein Dauerstents und Polymerstents; Legierungsbeispiele sind AZ 31, AZ61, AZ63, AZ80, AZ91, AZ92, WE54, EA55, EA65)
- Lipid mit zusätzlichem eingebettetem pH-Puffer (z.B. Calciumcarbonat, Natriumhydrogenphosphat, Calciumphosphate, Natriumbicarbonat usw.)
- Andere Lipide und flüssige Trägerstoffe außer Sojabohnenöl, z.B. Cholesterin, gesättigte Fette, Ölmischungen, Fettsäuren, Ester von Fettsäuren, Phospholipide, Mono-, Di-, Triacylglyceride usw.
- Beispiele für die kristallinen Phasen von Calciumphosphat (ohne einzuschränken): Mono-CaP-Monohydrat (MCPM) Ca(H₂PO₄)₂ · H₂O, Di-CaP-Dihydrat (DCPD) CaH-CaHPO₄ · 2H₂O, Octa-CaP (OCP) Ca₈H₂(PO₄)₆ · 5H₂O, Tri-CaP(TCP)Ca₃(PO₄)₂ (· 2H₂O) Hydroxyapatit (OHAp) Ca₁₀(PO₄)₆ (OH)₂, Tetra-CaP (TTCP) Ca₄(PO₄)₂O; Fluorapatit; Chlorapatit; kristallines Wachstum kann in vielen verschiedenen Formen auftreten, z.B. als Nadeln, Kugeln oder ebene Schichten.

Es folgt ein Beispiel für den Herstellungsprozess für Stents:
1. Reinigen der AMS-Oberfläche,
2. Hydroxyapatit in Elektrolytlösung auf der Stentoberfläche abscheiden (Plasmaverarbeitung, Tauchbeschichten, Plasmaspritzprozesse, chemische Aufdampfprozesse, Sinterprozesse, elektrochemische Prozesse, Vorkorrosion der AMS-Oberfläche, z.B. in künstlich erzeugtem Plasma als weitere Möglichkeit),
3. Trocknen,
4. Tauchbeschichten des Stents in Lösung, die aus Trägersubstanz/Medikament plus Lösungsmittel besteht,
5. Trocknen des Lösungsmittels,

Hintergrundinformationen zur alternativen Herstellung einer Calciumphosphat- (CaP04)-Oberfläche: Vorreaktion der Implantatsoberfläche in einer physiologischen Lösung oder ähnlichen Lösungen: Schichten, die auf diese Weise erzeugt wurden, enthalten bestimmte Mengen oder Spuren des früheren Grundmaterials, das umgewandelt wurde. Die alternative Herstellung kann auch darin bestehen, dass durch eine in situ-Reaktion (Vorreaktion, Reaktion des ursprünglichen Materials des Stentkörpers) und die zusätzliche Beschichtung mit einem der oben genannten Prozesse eine erste Schicht erzeugt wird.

## Patentansprüche

1. Medizinisches Implantat, das aus einem Grundmaterial hergestellt ist, welches aus einem biologisch abbaubaren Metall und/oder einer biologisch abbaubaren Metalllegierung besteht, wobei das Implantat eine Beschichtung enthält, die aus folgendem hergestellt ist:
- kristallines Calciumphosphat
- und/oder amorphes Calciumphosphat.

2. Implantat nach Anspruch 1, wobei die Beschichtung zumindest Spuren oder Ausscheidungen des Grundmaterials des Implantats enthält.

3. Implantat nach Anspruch 1 oder 2, wobei das Grundmaterial des Implantats Magnesium oder eine Magnesiumlegierung ist.

4. Implantat nach Anspruch 3, wobei die Magnesiumlegierung ein Legierungselement von bis zu 50 Atomprozent aus der Gruppe bestehend aus Calcium, Zink, Mangan, Lithium, Eisen oder bis zu 50 Atomprozent einer Kombination derselben enthält.

5. Implantat nach Anspruch 3 oder 4, wobei die Magnesiumlegierung zusätzlich bis zu 20 Atomprozent Yttrium oder andere Seltenerdelemente enthält.

6. Implantat nach Anspruch 1 oder 2, wobei die Beschichtung eine Schichtstruktur aufweist.

7. Implantat nach Anspruch 6, wobei die Beschichtung mindestens eine kristalline Schicht und/oder amorphe Schicht hat, wobei die kristalline Schicht und/oder die amorphe Schicht eine Schichtdicke von 0,1 bis 5 Mikrometern hat.

8. Implantat nach Anspruch 7, wobei die kristalline Schicht die gesamte Oberfläche des Implantats bedeckt und die amorphe Schicht die kristalline Schicht bedeckt.

9. Implantat nach Anspruch 7 oder 8, wobei die amorphe Schicht Poren mit einer Porengröße zwischen 0,02 und 50 Mikrometern hat.

10. Implantat nach Anspruch 7 oder 8, wobei die amorphe Schicht aus Hydroxyapatit besteht und Poren mit einer Porengröße zwischen 0,02 und 1 Mikrometer hat.

11. Implantat nach einem der Ansprüche 9-10, wobei die Poren der amorphen Schicht mit einem oder mehreren Lipiden gefüllt sind, vorzugsweise Fettsäure, Mono-, Di- oder Triacylglyceriden, Wachsen, Phospholipiden, Sphingolipiden, Steroiden und abbaubaren Polymeren, die einen nichtsauren Zerfallsmechanismus zeigen.

12. Implantat nach Anspruch 11, wobei ein Medikament und/oder ein pH-Puffer in die Lipide eingebettet ist, insbesondere ein antiproliferatives und/oder entzündungshemmendes Medikament.

13. Implantat nach einem der vorherigen Ansprüche, wobei das Implantat ein Stent ist.
